# EUROPEAN PATENT SPECIFICATION

(11) **EP 2 678 441 B1**
(45) Date of publication and mention of the grant of the patent: **18.10.2017**
(21) Application number: 12704842.9
(22) Date of filing: 22.02.2012
(51) Int. Cl.: C12Q 1/68

(54) **SINGLE NUCLEOTIDE POLYMORPHISMS IN THE PROMOTER OF VEGFA GENE AND THEIR USE AS PREDICTIVE MARKERS FOR ANTI-VEGF TREATMENTS**
EINZELNUKLEOTID-POLYMORPHISMEN IM PROMOTOR DES VEGFA-GENS UND VERWENDUNG DAVON ALS PRÄDIKTIVE MARKER FÜR ANTI-VEGF-BEHANDLUNGEN
POLYMORPHISMES DE NUCLÉOTIDE SIMPLE DANS LE PROCÉDÉ DU GÈNE VEGFA ET LEUR UTILISATION EN TANT QUE MARQUEURS PRÉDICTIFS POUR DES TRAITEMENTS ANTI-VEGF

(30) Priority: 23.02.2011 EP 11305188
(43) Date of publication of application: 01.01.2014
(73) Proprietor: SANOFI, 75008 Paris (FR)
(72) Inventor: CHIRON BLONDEL, Marielle, F-75008 Paris (FR); COUSIN, Emmanuelle, F-75008 Paris (FR); DELEUZE, Jean-François, F-75008 Paris (FR); DREYMANN, Jennifer, F-75008 Paris (FR); MACE, Sandrine, F-75008 Paris (FR)
(74) Representative: Bouvet, Philippe
(86) International application number: PCT/EP2012/053026
(87) International publication number: WO 2012/123227

(56) References cited:
- WO-A2-2009/073540
- WO-A2-2010/054110
- WO-A2-2010/124264
- FORMICA V ET AL: "VEGF polymorphisms as predictors of bevacizumab efficacy in metastatic colorectal cancer", EUROPEAN JOURNAL OF CANCER. SUPPLEMENT, PERGAMON, OXFORD, GB, vol. 7, no. 4, 1 October 2009 (2009-10-01) , page 17, XP026692181, ISSN: 1359-6349, DOI: DOI:10.1016/S1359-6349(09)72163-9 [retrieved on 2009-10-01]
- VINCENZO FORMICA ET AL: "Predictive value of VEGF gene polymorphisms for metastatic colorectal cancer patients receiving first-line treatment including fluorouracil, irinotecan, and bevacizumab", INTERNATIONAL JOURNAL OF COLORECTAL DISEASE ; CLINICAL AND MOLECULAR GASTROENTEROLOGY AND SURGERY, SPRINGER, BERLIN, DE, vol. 26, no. 2, 29 December 2010 (2010-12-29), pages 143-151, XP019875249, ISSN: 1432-1262, DOI: DOI:10.1007/S00384-010-1108-1
- A ABAJO ET AL: "Dose-finding study and pharmacogenomic analysis of fixed-rate infusion of gemcitabine, irinotecan and bevacizumab in pretreated metastatic colorectal cancer patients", BRITISH JOURNAL OF CANCER, vol. 103, no. 10, 9 November 2010 (2010-11-09), pages 1529-1535, XP055000421, ISSN: 0007-0920, DOI: 10.1038/sj.bjc.6605908
- SMERDEL M P ET AL: "The predictive value of serum VEGF in multiresistant ovarian cancer patients treated with bevacizumab", GYNECOLOGIC ONCOLOGY, ACADEMIC PRESS, LONDON, GB, vol. 118, no. 2, 1 August 2010 (2010-08-01), pages 167-171, XP027117449, ISSN: 0090-8258 [retrieved on 2010-05-14]
- LOUPAKIS F ET AL: "6115 VEGF gene polymorphisms in the prediction of benefit from first-line FOLFIRI plus bevacizumab (BV) in metastatic colorectal cancer (mCRC) patients (pts)", EUROPEAN JOURNAL OF CANCER. SUPPLEMENT, PERGAMON, OXFORD, GB, vol. 7, no. 2, 1 September 2009 (2009-09-01), page 357, XP026690016, ISSN: 1359-6349, DOI: DOI:10.1016/S1359-6349(09)71210-8 [retrieved on 2009-09-01]
- BRANTLEY ET AL: "Association of Complement Factor H and LOC387715 Genotypes with Response of Exudative Age-Related Macular Degeneration to Intravitreal Bevacizumab", OPHTHALMOLOGY, J. B. LIPPINCOTT CO., PHILADELPHIA, PA, US, vol. 114, no. 12, 27 November 2007 (2007-11-27), pages 2168-2173, XP022382053, ISSN: 0161-6420, DOI: DOI:10.1016/J.OPHTHA.2007.09.008
- PASQUALETTI GIUSEPPE ET AL: "Vascular endothelial growth factor pharmacogenetics: a new perspective for anti-angiogenic therapy", PHARMACOGENOMICS, ASHLEY PUBLICATIONS, GB, vol. 8, no. 1, 1 January 2007 (2007-01-01) , pages 49-66, XP008128080, ISSN: 1462-2416, DOI: DOI:10.2217/14622416.8.1.49
- RAKESH K. JAIN ET AL: "Biomarkers of response and resistance to antiangiogenic therapy", NATURE REVIEWS CLINICAL ONCOLOGY, vol. 6, no. 6, 1 June 2009 (2009-06-01), pages 327-338, XP055000425, ISSN: 1759-4774, DOI: 10.1038/nrclinonc.2009.63
- BROGAN J J ET AL: "NOVEL POLYMORPHISMS IN THE PROMOTER AND 5' UTR REGIONS OF THE HUMANVASCULAR ENDOTHELIAL GROWTH FACTOR GENE", HUMAN IMMUNOLOGY, NEW YORK, NY, US, vol. 60, no. 12, 1 January 1999 (1999-01-01), pages 1245-1249, XP000979038, ISSN: 0198-8859, DOI: DOI:10.1016/S0198-8859(99)00132-9
- KETAO JIN ET AL: "Aflibercept (VEGF Trap): one more double-edged sword of anti-VEGF therapy for cancer?", CLINICAL AND TRANSLATIONAL ONCOLOGY, vol. 12, no. 8, 1 August 2010 (2010-08-01) , pages 526-532, XP055000427, ISSN: 1699-048X, DOI: 10.1007/s12094-010-0550-4
- QUINCY SIU-CHUNG CHU: "Aflibercept (AVE0005): An alternative strategy for inhibiting tumour angiogenesis by vascular endothelial growth factors", EXPERT OPINION ON BIOLOGICAL THERAPY, ASHLEY, LONDON, GB, vol. 9, no. 2, 1 January 2009 (2009-01-01) , pages 263-271, XP009148906, ISSN: 1471-2598, DOI: 10.1517/14712590802666397

## Description

The present invention concerns anti-angiogenic therapies and more particularly relates to a group of patients bearing specific alleles of single nucleotide polymorphisms (SNPs) in the promoter sequence of VEGFA gene, found to be more responsive to anti-VEGF treatments.

Anti-angiogenic therapies have improved the clinical outcome of selected tumors by inhibiting tumor growth via restriction of new blood vessel formation. As the tumor grows, the lack of sufficient blood supply creates a hypoxic environment, stimulating the release of factors like hypoxia inducible factor (HIF)-1α, which induces angiogenesis by activating the transcription of vascular endothelial growth factor (VEGF) [Ferrara, N. et al. (2002) VEGF and the quest for tumor angiogenesis factors. Nat. Rev. Cancer. 2:795-803].

VEGFA and its receptors are frequently over-expressed in human tumors, including the breast, non-small cell lung, colorectal, and prostate cancers. VEGFA has been more particularly implicated with poor prognosis in breast cancer. Numerous studies show a decreased overall survival and disease-free survival in those tumors over-expressing VEGFA. Although the exact mechanism of action of VEGFA in the progression of tumors remains unclear, the expression of VEGFA is likely to play a central role in the vascularization of the tumors.

The broad term 'VEGFA' covers a number of proteins from two families, that result from alternate splicing of mRNA from a single, 8-exon, *VEGFA* gene. The *VEGFA* gene is located on chromosome 6 at location 6p21.1. Its coding region spans approximately 14 kilobases and consists of eight exons [Tischer, E. et al. The human gene for vascular endothelial growth factor. Multiple protein forms are encoded through alternative exeon splicing (1991) J. Biol. Chem. 266:11947-954]. Numerous SNPs are present in the promoter, 5'-, and 3'-untranslated regions (UTR) of *VEGFA.*

VEGFA and associated signalling pathways have been the target of many new anticancer drugs. In particular, optimized ligands of VEGFA, referred to as anti-VEGF proteins, capable of inhibiting or reducing the biological activity of VEGFA are currently being developed. These anti-VEGF proteins can be derived from natural or artificial proteins displaying some affinity with VEGFA, such as anti-VEGFA antibodies or VEGFA receptors. Examples of such anti-VEGF proteins, which have been made so far available for commercialization or clinical trials, are Bevacizumab (Avastin) [Ferrara N. et al. (2004) Discovery and development of Bevacizumab, an anti-VEGF antibody for treating cancer, Nature Reviews 3:391-400], Ranibizumab (Lucentis) and Aflibercept (VEGF-trap) [Holash, J. (2002) VEGF-Trap: A VEGF blocker with potent antitumor effects, PNAS, 99(17):11393-98].

Aflibercept is a VEGFA polypeptide ligand comprised of segments of the extracellular domains of human vascular endothelial growth factor receptors 1 (VEGFR1) and 2 (VEGFR2) fused to the constant region (Fc) of human IgG1 with anti-angiogenic activity. This protein functions as a soluble decoy receptor. It binds to pro-angiogenic vascular endothelial growth factors (VEGFAs), thereby preventing VEGFAs from binding to their cell receptors. Disruption of the binding of VEGFAs to their cell receptors results in the inhibition of tumor angiogenesis, metastasis, and ultimately in tumor regression.

Anti-VEGF molecules have been also found to be useful for treating macular degeneration as counteracting the effects of VEGFA may provide a significant therapeutic effect in patients suffering from this disease. Age-related Macular Degeneration (AMD) is a leading cause of acquired blindness. In AMD, new blood vessels grow beneath the retina and leak blood and fluid. This leakage causes disruption and dysfunction of the retina, creating blind spots in central vision, and it can account for blindness in such patients.

Anti-VEGF molecules have shown promising efficacy in preclinical and clinical studies [Rakesh K.J. et al. (2006) Lessons from Phase III clinical trials on anti-VEGF therapy for cancer, Nature Clinical Practice Oncology, 3:24-40]. However, they are often very expensive to produce, and some patients experience drug resistance, limited activity, and severe toxicities. There is therefore a strong need for identification of markers enabling *a priori* selection of patients who are likely to benefit from these agents.

Several studies have examined the role of SNPs in the *VEGFA* gene as predictive and prognostic markers for major solid tumors. However, these studies resulted in conflicting reports [Jain L. et al. (2009) Mol. Cancer. Ther. 8(9):2496-2508]. Excepting the -1154G>A SNP, which was acknowledged as being correlated with a significant reduced risk of prostate cancer, no other VEGFA SNPs have been identified as being significantly associated with a lower or higher incidence of cancer.

Another study by Schneider et al. [J. Clin. Oncol. (2008) 26:4672-4678] found that the VEGFA -2578AA genotype is associated with a superior median overall survival in comparison to patients bearing -2578C alleles, in metastatic breast cancer patients treated with Bevacizumab. Formica et al (EUROPEAN JOURNAL OF CANCER. SUPPLEMENT, vol. 7, no. 4, 2009, page 17) and Formica et al (INTERNATIONAL JOURNAL OF COLORECTAL DISEASE; vol. 26, no. 2, 2011, pages 143-151) disclose the results of a study evaluating the influence of (VEGF) gene polymorphisms on the efficacy of the bevacizumab in metastatic colorectal cancer patients. Several polymorphisms are detected. Patients having the C allele at position -2578 exhibit a longer median progression free survival.
Abajo et al. (BRITISH JOURNAL OF CANCER, vol. 103, no. 10, 2010, pages 1529-1535) discloses VEGF promoter polymorphisms and genotypes that correlate with a longer median time to progression in colorectal cancer patients treated with a combination of gemcitabine, irinotecan and bevacizumab.
WO 2009/073540 discloses the results of a Phase III trial. Paclitaxel or bevacizumab and paclitaxel are administered to women with previously untreated metastatic breast cancer.
VEGF -2578 AA genotype and the VEGF -1154 AA genotypes tend to show a favorable overall survival for patients in the combination arm.
Pasqualetti et al (PHARMACOGENOMICS, vol. 8, no. 1, 2007, pages 49-66) is a review of pharmacogenetics of anti-angiogenic therapy. They mention the need to study the relationship between efficacy of these therapeutics and VEGF-A gene haplotypes and polymorphisms.

Thus, to date, it remains difficult to predict as whether a patient developing a cancer or macular degeneration is likely to be responsive to anti-VEGF treatment.

The present invention discloses a study which unexpectedly shows that patients bearing some specific mutations in the promoter sequence of *VEGFA* gene are more responsive than others to treatments based on anti-VEGF proteins, especially to Aflibercept. These mutations correspond to polymorphisms (SNPs) naturally found in the human world population corresponding to the alleles referred to as: -2578C, -2549del, -1498T and - 1190G.

These patients appear to form a group, which is more responsive to anti-VEGF treatments, and, therefore, is likely to have a better treatment outcome.

Based on these findings, the invention provides a diagnostic method, which consists of a genetic test that can be performed on blood samples, to determine which patients bear these SNPs and should be treated using anti-VEGF molecules.

The invention also proposes to address anti-VEGF treatment primarily to patients bearing at least one of the alleles -2578C, -2549del, -1498T and - 1190G.
**Figures 1A to 1D****:** Representation of the promoter sequence of the *VEGFA* gene, showing the respective polymorphisms -2578C, -2549del, -1498T and - 1190G in bold characters.
**Figures 2A to 2D****:** Representation of the promoter sequence of the *VEGFA* gene. This sequence is considered in the context of the present invention as the reference or wild-type polynucleotide sequence of the promoter of VEGFA.

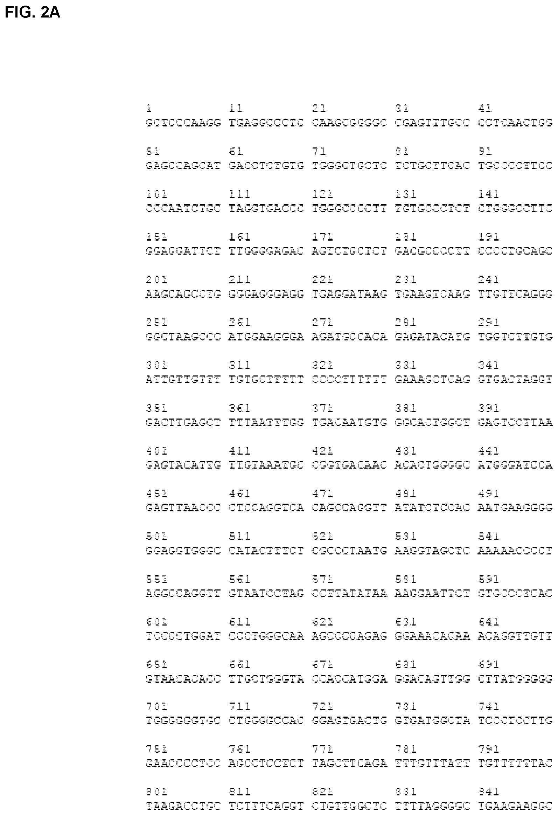

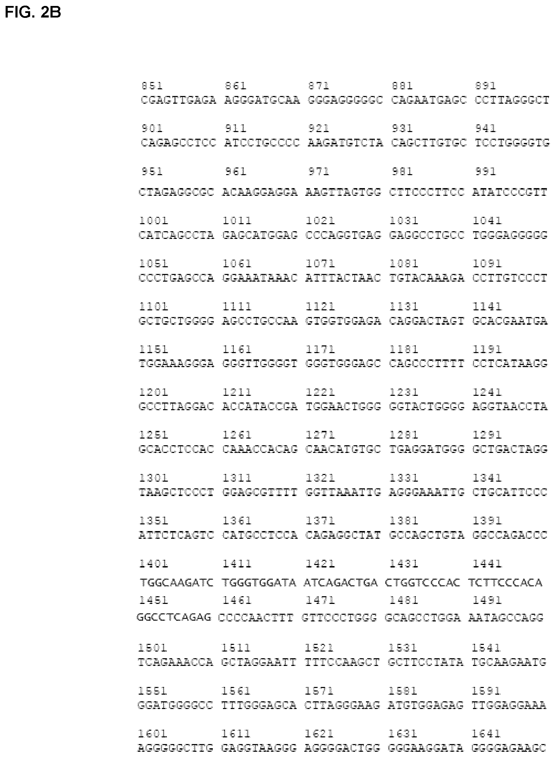

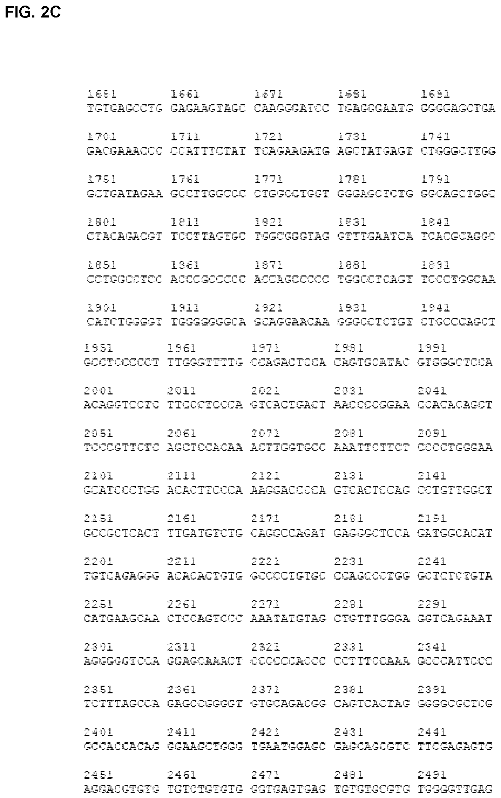

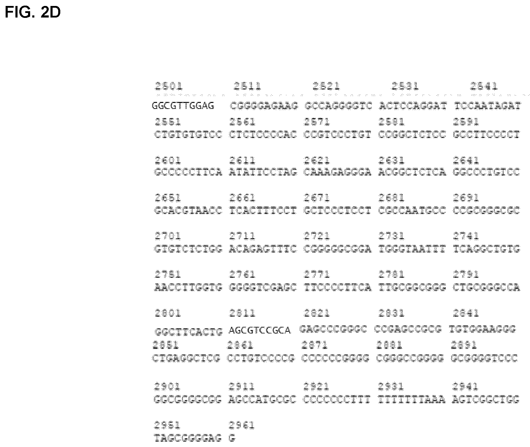

The present invention has therefore for object a general diagnostic method for determining whether a patient is likely to offer a positive response to aflibercept cancer therapy, wherein said method comprises the step of detecting the presence of at least one of the polymorphisms -2578C, - 2549del, -1498T and -1190G in the promoter sequence of the gene encoding VEGFA, such as the sequence of Figure 2, isolated from said patient. The nucleotide polymorphism may correspond to a single mutation (SNP), a sequence deletion (del), insertion (ins), duplication, in comparison to the wild type sequence of Figure 2. Such a polymorphism is detectable by any standard methods known in the art.

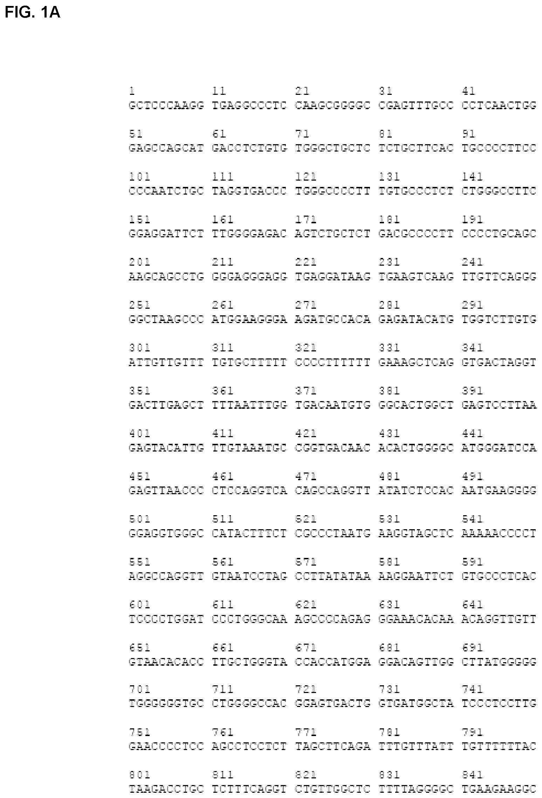

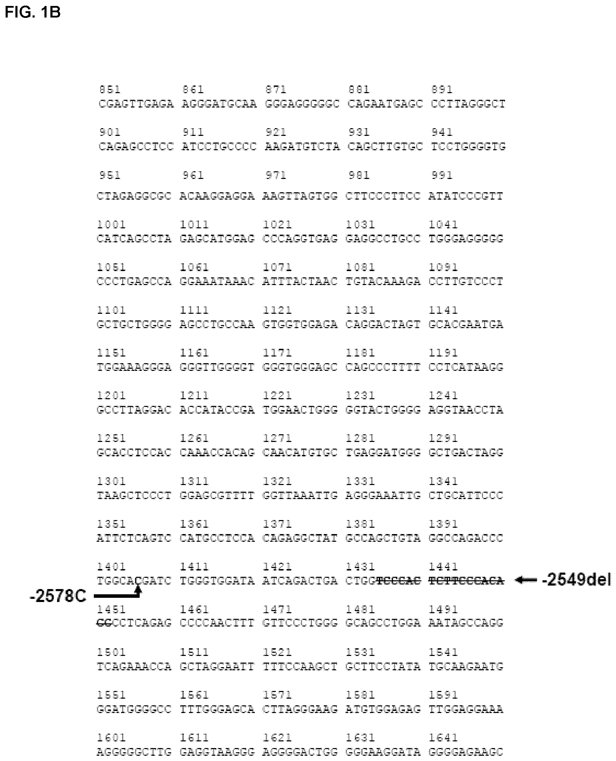

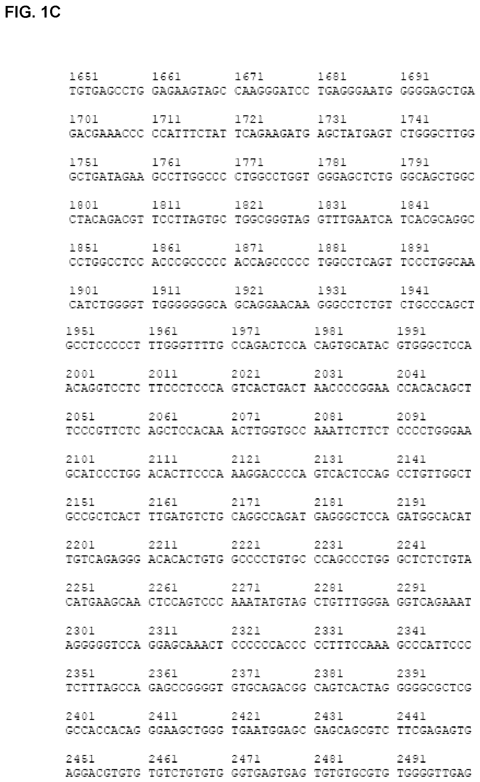

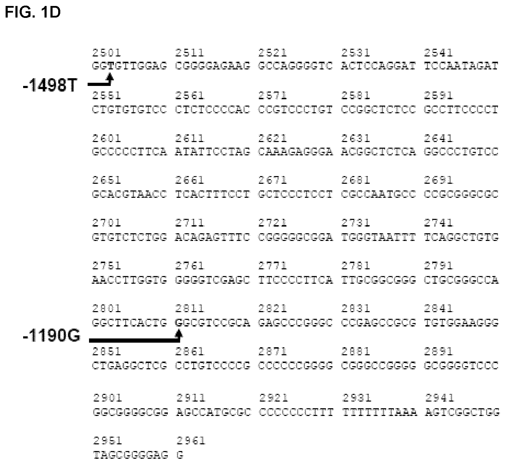

The numbering of the polymorphisms corresponds to the positioning of the mutation or deletion relative to the first nucleotide of the translation initiation codon of the *VEGFA* gene. It is preceded by a minus sign to indicate that it is positioned before said first nucleotide. The letters A, T, C, and G correspond respectively to the nitrogenous bases adenine, thymine, cytosine and guanine. The abbreviation "del", with respect to -2549del, means a deletion of one or several nucleotide base(s) initially present at the position - 2549 before the "ATG" site of transcription. In an embodiment this deletion corresponds to one or several bases from the positions 1435 to 1452 of the sequence as shown in bold in Figure 1.

The sequence of Figure 2 represents the main part of the promoter sequence of the *VEGFA* gene. The whole *VEGFA* gene sequence is available from the human genome database (Chromosome 6 contig GRCh37.p2 - ref. NCBI: NT_007592). This sequence is considered in the context of the present invention as the reference or wild-type polynucleotide sequence of the promoter of VEGFA.

The sequence of Figure 1 represents the promoter sequence of *VEGFA* gene (same region as SEQ ID NO.1) including the four polymorphisms -2578C, -2549del, -1498T and -1190G altogether, as a allele sequence according to the invention.

When the polymorphisms are detected independently from each other, the respective alleles so detected are simply referred to as -2578C, -2549del, -1498T or -1190G.

Table 1 shows the correspondence between the notation used for the allele mutations in the present patent application, and other equivalent notations taking into account the position of the bases with respect to the transcription initiation site of *VEGFA.* The changes induced by the polymorphisms in comparison to the sequence of Figure 2 and the reference numbers for each SNP in the NCBI database (www.ncbi.nlm.nih.gov/snp) are also indicated.

**Table I**

| SNP notations | | | |
|---|---|---|---|
| sequence of Figure 2>1 | (VEGFA gene) NT_007592 | | NCBI |
| Corresponding mutations referred to in sequence of Figure 2 | Position numbered from translation initiation site | Position numbered from transcription initiation site | SNP database reference |
| A > C (1406) | - 2578 C | -1540C | rs699947 |
| ins > del (1435 -1452) | - 2549 del | - 1511 del | rs34357231 |
| C > T (2502) | -1498T | - 460 T | rs833061 |
| A > G (2811) | -1190G | - 152 G | rs13207351 |

In order to perform the diagnostic method according to the invention as an *in-vitro* method, the polynucleotide DNA sequence is primarily isolated from the patient by routine procedures. It can be isolated from a blood sample.

The patients to be diagnosed are generally patients who were primarily diagnosed for cancer, macular degeneration or any other disease susceptible to be treated using anti-VEGF therapy. Breast cancer, non-small cell lung, colorectal and prostate cancers have been so far those known in the art as being more susceptible to be treated by anti-VEGF therapy.

According to the present invention, anti-VEGF therapy can be performed with anti-VEGF proteins, which are can be proteins derived from VEGF receptors or from antibodies directed against VEGF proteins, more particularly anti-VEGFA proteins.

It shall be noted that the alleles -2578C, -2549del, -1498T and -1190G detectable according to the invention are usually found together in the same patient genomes, as they are statistically linked (unbalanced allele distribution). Accordingly, when one of these alleles is found in one patient's genome, the others are likely to be also present in the VEGFA promoter sequence of that patient, as represented, for instance, in sequence of Figure 1.

It is noteworthy that the above SNPs have a germinal origin and thus generally do not result from mutations occurring during the process of tumor transformation. The inventors have established that in 99 % of the analyzed tumors, the alleles bearing the SNPs were present in non tumor cells surrounding the tumor cells. These SNPs being from germinal origin, the diagnostic method of the invention can be performed directly from blood sample, rather than from tumor samples, which constitutes a much less invasive diagnosis method.

Patients bearing one of the alleles -2578C, -2549del, -1498T and - 1190G in their VEGFA gene promoter sequence have also been found to constitute a group of patients that are more responsive to anti-VEGF treatments.

Particularly significant results were obtained with respect to the detection of the allele -2578C.

According to an embodiment of the invention, all four alleles referred to above can be sought after in the patient's genome as a SNP signature. Detecting two, three or four of these alleles allows one to obtain a more reliable diagnosis, than one individual allele. For example, the inventors have observed among non-small cell lung cancer patients a significant association between the presence of the alleles -2578C, -2549del and -1498T altogether, and a stabilisation over more than 120 days for this disease upon anti-VEGF treatment.

The present invention may be carried out using specific probes or primers designed to detect the above alleles within the genome of patients. The amplification techniques, which can be applied to the crude DNA preparations from the patient's biological samples, in particular from patients' blood, are those well known in the art. For example, PCR may be performed using primers differentiating the sequences in which alleles like the sequence of Figure 1, from a VEGFA promoter reference sequence like the sequence of Figure 2, where none of these alleles are present.

The probes or primers according to the invention can be polynucleotide sequences comprising the following polynucleotide sequences SEQ ID NO.1, SEQ ID NO.2, SEQ ID NO.3 or SEQ ID NO.4.

According to an embodiment of the invention, the detection is performed by PCR using the following PCR sequences:
- SEQ ID NO.1 (forward primer): CTGGGGAGCCAAGTGG;
- SEQ ID NO.2 (reverse primer): CCAGCACTAAGGAACGTCTGTAGGC.
The above sequences are particularly adapted to amplify and/or sequence the alleles -2578C and -2549del.
- SEQ ID NO.3 (forward primer): GCGAGCGTCTTCGAGAG;
- SEQ ID NO.4 (reverse primer): CCGGTCCACCTAACCGCTGC.
The above sequences are particularly adapted to amplify and/or sequence the alleles -1498T and -1190G.
Another object of the invention is a method for treating a human subject afflicted with, or at risk of developing, an angiogenesis-related disease or disorder, wherein said method comprises the step of:
(a) detecting in a nucleic acid sample from the subject the presence of at least one single nucleotide polymorphism (SNP) in the promoter sequence of the gene encoding VEGFA (sequence of Figure 2); and
(b) administering to said patient said anti-VEGF therapy if said SNP is present in said subject.
The invention relates furthermore to a method for selecting therapy for a human subject afflicted with, or at risk of developing, an angiogenesis-related disease or disorder, wherein said method comprises the step of:
(a) detecting in a nucleic acid sample from the subject the presence of at least one single nucleotide polymorphism (SNP) in the promoter sequence of the gene encoding VEGFA (sequence of Figure 2); and
(b) selecting for said a patient an anti-VEGF therapy if said SNP is present in said subject.

The invention has for further object a diagnostic kit, which allows one to perform the diagnostic method as previously described, in view of detecting patients that are more responsive to anti-VEGF therapy.

Such a diagnostic kit can comprise a set of primers, at least one of which comprises one of the sequences SEQ ID NO.1, SEQ ID NO.2, SEQ ID NO.3 and SEQ ID NO.4.

This kit may further comprises enzymes with polymerase activity, restriction enzymes, or marking agents helping to detect the presence of the above polymorphisms in patient's genomic DNA.

The above methods and kits aim to detect patients bearing the above polymorphisms or alleles in their genomes.

As per the experimental data collected by the inventors, the patients bearing at least one of the above alleles form a group of patients, distinct from the general population, which is particularly responsive to anti-VEGF therapy. The health condition of these patients is thus likely to be improved by an anti-VEGF treatment.

The diagnostic method according to the invention is therefore particularly intended to select patients to benefit anti-VEGF therapy for treating cancer, more specifically breast, non-small cell lung, colorectal or prostate cancer. It is also intended to select patients to benefit anti-VEGF therapy for treating macular degeneration.

By anti-VEGF therapy is meant any therapeutic treatment targeting a VEGF protein in order to lower or inhibit its biological activity.

A patient is deemed responsive to anti-VEGF therapy when such treatment improves his health condition with respect to the disease to be treated.

In the case of cancer, for instance, this may consist of reducing tumor growth. In the case of macular degeneration, this may be a stabilization of retina tissues.

In the present case, responsiveness was observed upon treatment of this group of patients using Aflibercept, a VEGF-trap molecule belonging to a class of molecules acting as anti-VEGFA ligands, i.e. inhibitor molecules binding to VEGFA. Thus, it is highly likely that the newly identified group of patients will be also responsive to other anti-VEGFA ligands, more particularly protein ligands derived from anti-VEGFA antibodies or receptors, such as Bevacizumab (Avastin) and Ranibizumab (Lucentis).

Without being bound by theory, the inventors have made the hypothesis that the mutations in the promoter of *VEGFA* may reduce the expression of VEGFA, thus improving the ratio VEGFA/anti-VEGFA molecules, which would make anti-VEGF treatment even more efficient. However, more experimental work would need to be conducted to fully determine the exact mechanisms underlying the effects of the SNPs on the anti-VEGF treatments.

In this context, the present invention also provides a method of treatment, according to which an anti-VEGF ligand is used for the treatment of cancer, especially breast and lung cancer treatment, in a group of patients bearing at least one allele of the promoter sequence of the *VEGFA* gene including -2578C, -2549del, -1498T or -1190G. Such a ligand can be an anti-VEGFA protein. In an embodiment it is Aflibercept,

This group of patients is defined by those patients in whom the above polymorphisms or alleles can be detected according to the diagnostic method of the invention. These patients offer a better response to anti-VEGF therapy in comparison to the general population.

Similarly, an anti-VEGF ligand as defined above, and more especially Ranibizumab (Lucentis), is also useful for the treatment of macular degeneration in a group of patients bearing at least one allele of the promoter sequence of the *VEGFA* gene including -2578C, -2549del, -1498T or - 1190G.

The following example is given to illustrate the invention in a non limitative manner.

### EXAMPLE

### Material and Methods

Crude DNA was extracted from full blood of patients having been treated with Aflibercept during clinical assays held by Sanofi-Aventis for evaluating the efficacy of this product against different cancers (lung, ovary and breast). This DNA extraction was made using the general method by Sambrook, J. [Molecular Cloning (Third Edition) (2001) Cold Spring Harbour Laboratory Press, N.Y.].

SNP -2578C/A was sequenced by the classical method by Sanger et *al.* on :
- 92 Caucasian individuals from the Coriell Institute to determine the frequency of these SNPs in a general population of healthy patients.
- 131 DNA tumor extracts (lung, ovary, breast) as well as 131 DNA samples extracted from the margin of the same tumors, to confirm the germinal origin of the genetic variants.
- 31 samples from patients pre-clinically treated with Aflibercept, to identify the possible link between these SNPs and the activity of Afl ibercept.
- 47 DNA extracts from blood from patients having lung cancer.
- 145 DNA extracts from blood from ovary cancer patients, the two latter analyses aiming to validate an association between the SNPs and an improved clinical activity of Aflibercept. The different VEGFA polymorphisms -2578C/A, -2549del/ins, -1498T/C and -1190G/A are described under the references rs699947, rs34357231, rs833061 and rs13207351 in the NCBI database dbSNP (see Table 1). Fig.1 illustrates an allele including 2578C, -2549del, -1498T and -1190G.

The following primers were used for both PCR and sequencing:
In order to amplify by PCR and sequence the alleles -2578C and -2549del:
   - SEQ ID NO.1 (forward primer): CTGGGGAGCCAAGTGG;
   - SEQ ID NO.2 (reverse primer): CCAGCACTAAGGAACGTCTGTAGGC.
In order to amplify by PCR and sequence the alleles -1498T and -1190G:
   - SEQ ID NO.3 (forward primer): GCGAGCGTCTTCGAGAG;
   - SEQ ID NO.4 (reverse primer): CCGGTCCACCTAACCGCTGC.

Genomic DNA (2 µl at 10 ng/µl) was used as template for PCR amplification. 1 µl of 10 µM forward primer, was added to 1 µl of 10 µM reverse primer and Promega 2 X mix (12 µl; Promega, Madison, Wisconsin, USA) for a final reaction volume of 20 µl. PCR conditions were as follows: 95°C for 3 min, followed by 2 cycles 95°C for 30 sec and 70°C for 30 sec, 2 cycles 95°C for 30 sec and 65°C x 30 sec, 2 cycles 95°C for 30 sec and 60°C for 30 sec, 40 cycles 95°C for 30 sec and 56°C for 30 sec and 72°C x 30 sec, ending with 72°C for 10 min.

The purified PCR products (2 µl), which were obtained were used as template for sequencing reactions. 1 µl of 10 µM primer was added to big Dye terminator sequencing mix (1.5 µl; Applied Biosystems, Foster City, USA) for a final reaction volume of 10 µl. Cycling conditions were performed as specified by the manufacturer. The sequencing reactions were purified on G50 Sephadex columns (Amersham). We analyzed the reactions on an ABI 3730 as recommended by the manufacturer. Assemblies and SNP identification were done using PHRED, PHRAP and POLYPHRED software.

### Results:

1. The C allele of the SNP -2578C/A tends to be associated with a preclinical response to Aflibercept.
2. The frequency of -2578C/A in a population deemed to be composed of healthy patients was found to be about 51 % for C allele (corresponding to sequence of Figure 1) and 49 % for A allele (corresponding to the sequence of Figure 2).
3. SNP -2578C was identified in both the tumor tissue and the marginal tissues, which demonstrates that this allele is present in the germinal cells, and is not due to tumor transformation. This was the case for 37 of 38 breast tumors, the 46 ovarian tumors, as well as the 47 lung tumors.
4. A significant association was found between -2578C and a positive response to Aflibercept with respect to lung cancer, as shown in Table 2. In these patients, -2578C was in complete association with the other alleles -2549del and -1498T (SNP bearing patient vs. non bearing patients (χ²) : p = 0.028 ; homogeneity test of allelic distribution (χ²): p = 0.044 ).

**Table 2**

| SNPs -2578C/A ; -2549del/ins ; -1498T/C | | |
|---|---|---|
| Genotype | <120 days | >120 days |
| CC/del-del/TT | 10 | 6 |
| AC/del-del/CT | 14 | 7 |
| AA/del-del/CC | 10 | 0 |

### Discussion:

Significant results were obtained when testing the association of the SNP marker -2578C with the response to Aflibercept treatment. The responding patients are those bearing one or two C alleles in position -2578, and the patients with a lower response to Aflibercept are those bearing two A alleles at this position.

Detecting the presence of such C alleles in patients should improve the benefit/risk balance of anti-VEGF treatment by treating only those patients susceptible to truly benefit from this treatment, and by avoiding exposing unnecessarily other patients to the toxic effects of anti-VEGF therapeutics.

The detection of -2578C seems to be sufficient to distinguish those patients responsive to anti-VEGF treatment. However, it may be put into combination with several other SNP markers, such as -2549del and -1498T, and also -1190G, which are in strong association with -2578C allele. This association thus forms a SNP signature that is helpful to identify more easily the patients likely to benefit from anti-VEGF treatment. Such a signature should not be only useful with respect to Aflibercept, but also for any VEGF inhibitors, in particular VEGFA ligands such as Avastin (protein derived from anti-VEGFA antibody Bevacizumab).

Furthermore, as -2578C allele is of germinal origin, it can be detected readily in a blood test by using standard genetic tests.

## Claims

1. Diagnostic method for determining whether a patient is likely to offer a positive response to aflibercept cancer therapy comprising the step of detecting the presence of at least one nucleotide polymorphism selected from -2578C, -2549del, -1498T or -1190G in the promoter sequence of the gene encoding VEGFA (Figure 2) isolated from said patient.

2. Diagnostic method according to claim 1, wherein said promoter sequence is isolated from the blood of said patient.

3. Diagnostic method according to any one of claims 1 or 2, wherein the allele -2578C is detected.

4. Diagnostic method according to any one of claims 1 to 3, wherein said alleles -2578C, -2549del, -1498T or -1190G are detected altogether.

5. Diagnostic method according to any one of claims 1 to 4, wherein said cancer to be treated is breast, non-small cell lung, colorectal or prostate cancer.

6. Diagnostic method according to claim 5, wherein said cancer is breast cancer.

7. Diagnostic method according to any one of claims 1 to 6, wherein the detection of said nucleotide polymorphism or allele is performed by hybridization of at least one primer, the sequence of which comprises SEQ ID NO.1, SEQ ID NO.2, SEQ ID NO.3 or SEQ ID NO.4.

8. Aflibercept for the treatment of cancer of a patient bearing at least one single nucleotide polymorphism selected from -2578C, - 2549del, -1498T or -1190G in the promoter sequence of the gene encoding VEGFA (Figure 2).

9. Aflibercept for the treatment of cancer of a patient according to claim 8 wherein the cancer is breast, non-small cell lung, colorectal or prostate cancer.

10. A method for selecting therapy for a human subject afflicted with, or at risk of developing, a cancer, wherein said method comprises the step of:
(a) detecting in a nucleic acid sample from the subject the presence of at least one single nucleotide polymorphism (SNP) selected from -2578C, -2549del, -1498T or -1190G in the promoter sequence of the gene encoding VEGFA (Figure 2) ; and
(b) selecting for said a patient an aflibercept therapy if said SNP is present in said subject.

## Patentansprüche

1. Diagnoseverfahren zur Bestimmung, ob ein Patient voraussichtlich eine positive Antwort auf die Aflibercept-Krebstherapie bietet, umfassend den Schritt, bei dem das Vorhandensein von mindestens einem Nukleotidpolymorphismus, ausgewählt aus -2578C, - 2549del, -1498T oder -1190G in der aus dem Patienten isolierten Promotorsequenz des Gens, das VEGFA codiert (Figur 2), erfasst wird.

2. Diagnoseverfahren nach Anspruch 1, wobei die Promotorsequenz aus dem Blut des Patienten isoliert wird.

3. Diagnoseverfahren nach einem der Ansprüche 1 oder 2, wobei das Allel -2578C erfasst wird.

4. Diagnoseverfahren nach einem der Ansprüche 1 bis 3, wobei die Allele -2578C, -2549del, -1498T oder - 1190G insgesamt erfasst werden.

5. Diagnoseverfahren nach einem der Ansprüche 1 bis 4, wobei es sich bei dem zu behandelnden Krebs um Brust-, nicht-kleinzelligen Lungen-, Kolorektal- oder Prostatakrebs handelt.

6. Diagnoseverfahren nach Anspruch 5, wobei es sich bei dem Krebs um Brustkrebs handelt.

7. Diagnoseverfahren nach einem der Ansprüche 1 bis 6, wobei das Erfassen des Nukleotidpolymorphismus oder -Allels durch Hybridisierung von mindestens einem Primer durchgeführt wird, dessen Sequenz SEQ ID NO: 1, SEQ ID NO: 2, SEQ ID NO: 3 oder SEQ ID NO: 4 umfasst.

8. Aflibercept zur Behandlung von Krebs eines Patienten, der mindestens einen Einzelnukleotidpolymorphismus, ausgewählt aus -2578C, - 2549del, -1498T oder -1190G in der Promotorsequenz des Gens, das VEGFA codiert (Figur 2), trägt.

9. Aflibercept zur Behandlung von Krebs eines Patienten nach Anspruch 8, wobei es sich bei dem Krebs um Brust-, nicht-kleinzelligen Lungen-, Kolorektal-oder Prostatakrebs handelt.

10. Verfahren zum Auswählen einer Therapie für ein menschliches Individuum, das von einem Krebs betroffen ist oder der Gefahr unterliegt, einen solchen zu entwickeln, wobei das Verfahren den Schritt umfasst:
(a) Erfassen in einer Nukleinsäureprobe aus dem Individuum die Anwesenheit von mindestens einem Einzelnukleotidpolymorphismus (SNP), ausgewählt aus - 2578C, -2549del, -1498T oder -1190G in der Promotorsequenz des Gens, das VEGFA codiert (Figur 2); und
(b) Auswählen einer Aflibercept-Therapie für den Patienten, wenn der SNP in dem Individuum vorhanden ist.

## Revendications

1. Procédé de diagnostic pour déterminer si un patient est susceptible de présenter une réponse positive à un traitement anticancéreux avec l'aflibercept comprenant l'étape de détection de la présence d'au moins un polymorphisme de nucléotide choisi parmi -2578C, -2549del, -1498T ou -1190G dans la séquence de promoteur du gène codant pour VEGFA (figure 2) isolé à partir dudit patient.

2. Procédé de diagnostic selon la revendication 1, dans lequel ladite séquence de promoteur est isolée à partir du sang dudit patient.

3. Procédé de diagnostic selon l'une quelconque des revendications 1 ou 2, dans lequel l'allèle -2578C est détecté.

4. Procédé de diagnostic selon l'une quelconque des revendications 1 à 3, dans lequel lesdits allèles -2578C, -2549del, -1498T ou -1190G sont détectés conjointement.

5. Procédé de diagnostic selon l'une quelconque des revendications 1 à 4, dans lequel ledit cancer devant être traité est un cancer du sein, du poumon non à petites cellules, colorectal ou de la prostate.

6. Procédé de diagnostic selon la revendication 5, dans lequel ledit cancer est un cancer du sein.

7. Procédé de diagnostic selon l'une quelconque des revendications 1 à 6, dans lequel la détection dudit polymorphisme de nucléotide ou allèle est effectuée par hybridation d'au moins une amorce, dont la séquence comprend SEQ ID NO. 1, SEQ ID NO. 2, SEQ ID NO. 3 ou SEQ ID NO. 4.

8. Aflibercept pour le traitement du cancer d'un patient comportant au moins un polymorphisme d'un seul nucléotide choisi parmi -2578C, -2549del, -1498T ou -1190G dans la séquence de promoteur du gène codant pour VEGFA (figure 2).

9. Aflibercept pour le traitement du cancer d'un patient selon la revendication 8, le cancer étant un cancer du sein, du poumon non à petites cellules, colorectal ou de la prostate.

10. Procédé de sélection d'une thérapie pour un sujet humain atteint de, ou à risque de développer, un cancer, ledit procédé comprenant l'étape de :
(a) détection dans un échantillon d'acide nucléique du sujet de la présence d'au moins un polymorphisme d'un seul nucléotide (SNP) choisi parmi -2578C, -2549del, -1498T ou -1190G dans la séquence de promoteur du gène codant pour VEGFA (figure 2) ; et
(b) sélection pour ledit patient d'une thérapie par aflibercept si ledit SNP est présent dans ladit sujet.
